Europäisches Patentamt

European Patent Office

Office européen des brevets

(11) Publication number: **0 211 022**
**B1**

(12) **EUROPEAN PATENT SPECIFICATION**

(45) Date of publication of patent specification: **11.04.90**

(21) Application number: **86900767.4**

(22) Date of filing: **21.01.86**

(86) International application number:
**PCT/EP86/00018**

(87) International publication number:
**WO 86/04336 31.07.86 Gazette 86/17**

(51) Int. Cl.⁵: **C 07 K 15/00, C 12 P 21/00, C 12 N 5/00, G 01 N 33/569, G 01 N 33/577, C 07 K 3/18 // C12N15/00 ,(C12P21/00, C12R1:91)**

(54) **MONOCLONAL ANTIBODIES AGAINST CORE PROTEINS OF LYMPHADENOPATHY-ASSOCIATED-VIRUSES.**

(30) Priority: **21.01.85 GB 8501473**

(43) Date of publication of application:
**25.02.87 Bulletin 87/09**

(45) Publication of the grant of the patent:
**11.04.90 Bulletin 90/15**

(84) Designated Contracting States:
**AT BE CH DE FR GB IT LI LU NL SE**

(56) References cited:
**EP-A- 138 667**

**Proc. Natl. Acad. Sci. USA, volume 82, August 1985 - F. Di Marzo Veronese et al. : "Monoclonal antibodies specific for p24, the major core protein of human T-cell leukemia virus type III", pages 5199-5202**

**Biological Abstracts/Reviews, Reports, Meetings, 1985 - F.V. Dimarzo et al.: "Monoclonal antibodies specific to human T cell leukemia virus III gag proteins"**
**Science, volume 225, 6 July 1984 - P.M. Feorino**

(73) Proprietor: **INSTITUT PASTEUR**
**25-28, rue du Docteur Roux**
**F-75724 Paris Cédex 15 (FR)**
(73) Proprietor: **CENTRE NATIONAL DE LA RECHERCHE SCIENTIFIQUE (CNRS)**
**15, Quai Anatole France**
**F-75008 Paris (FR)**

(72) Inventor: **CHASSAGNE, Jacques**
**3, rue de Leyrat**
**F-63670 Le Cendre (FR)**
Inventor: **VERRELLE, Pierre**
**Rue Guyot-Dessaigne**
**F-63730 Plauzat (FR)**
Inventor: **KLATZMANN, David**
**84, quai de Jemmapes**
**F-75010 Paris (FR)**
Inventor: **MONTAGNIER, Luc**
**21, rue de Malabry**
**F-92350 Le Plessis Robinson (FR)**
Inventor: **DIONET, Claude**
**27, La Rivière**
**F-63670 Le Cendre (FR)**
Inventor: **GLUCKMAN, Jean-Claude**
**70, bld. de Port Royal**
**F-75005 Paris (FR)**

Courier Press, Leamington Spa, England.

(56) References cited:
et al.: "Lymphadenopathy associated virus infection of a blood donor-recipient pair with acquired immunodeficiency syndrome", pages 69-72

Journal of Experimental Medicine, volume 159, April 1984 - T.J. Palker et al.:"Monoclonal antibodies against human Tcell leukemia-lymphoma virus (HTLV) p24 internal core protein", pages 1117-1131

(74) Representative: **Gutmann, Ernest et al**
**S.C. Ernest Gutmann - Yves Plasseraud 67,**
**boulevard Haussmann**
**F-75008 Paris (FR)**

## Description

The invention relates to monoclonal antibodies which recognize polypeptides, whether glycosylated or not, encoded by genomic RNA of lymphadenopathy-associated virus (LAV), or cloned DNA derived therefore, to the hybridomas secreting said antibodies and to a process for their preparation and finally to their uses. Such a virus has been disclosed in European patent application nr. 84.401834.1/0138667. The latter discloses that said retrovirus contains a p25 core protein which does not cross-react immunologically with the p24 protein of HTLV-I, a human T-lymphotropic Leukemia Virus. Monoclonal antibodies recognizing the HTLV-I p24 protein has been disclosed in the J. of Experimental Med. (1984), vol. 159, 1117—1131.

A retrovirus virtually identical to LAV and designated as HTLV-III is disclosed in Science (1984), vol. 225, 69—72.

A method for cloning DNA sequences of LAV has already been disclosed in British patent application nr. 84.23659 filed on September 19, 1984, in the European patent application nr. 85.401799 filed on September 17, 1985 and in the International Patent Application PCT/EP/85.00487 filed on September 18, 1985. Reference is hereafter also made to these applications as concerns subject matter in common with the further improvements to the invention disclosed herein.

The present application will also refer herein to the contents of the International application filed on October 18, 1985 PCT/EP 85.00548 on behalf of Institut Pasteur and the Centre National De La Recherche Scientifique. It is understood that the contents of the two preceding applications are entirely incorporated herein by reference.

More particularly the molecular cloning of both cDNA and integrated proviral forms of LAV have been reported. The recombinant phage clones were isolated from a genomic library of LAV-infected human T-lymphocytes DNA partially digested by HindIII. The insert of recombinant phage lambdaJ19 was generated by HindIII cleavage within the R element of the long terminal repeat (LTR). Thus each extremity of the insert contains one part of the LTR. The possibility of clustered HindIII sites within R has been eliminated by sequencing part of a LAV cDNA clone, pLAV 75, corresponding to this region. Thus the total sequence information of the LAV genome can be derivated from the λJ19 clone.

Using the M13 "shotgun" cloning and dideoxy chain termination method (Sanger et al., 1977), the nucleotide sequence of the λJ19 insert has been determined. The reconstructed viral genome with two copies of the R sequence is 9193 nucleotides long. The numbering system starts at the cap site of virion RNA. The entire sequence is shown in Figures 1a—1e of the International application, also enclosed herewith.

The present invention aims at providing for the more accurate identification of significant LAV proteins or glycoproteins and also at providing monoclonal antibodies against proteins and polypeptides carrying significant immunogenic sites or epitopes of the LAV virus proteins or glycoproteins.

The invention is more particularly concerned with monoclonal antibodies which recognize LAV core proteins or fragments thereof, particularly the p13, p18, p25 and p55 proteins.

The invention is thus more particularly concerned with and relates to monoclonal antibodies which recognize respectively:

—a p55 protein deemed to be encoded by the DNA sequence, extending from about nucleotide 336 up to about nucleotide 1650 of the LAVcDNA, which p55 protein is considered to contain aminoacid sequences corresponding to those of the core proteins p18 and p25 of the LAV virus;

—a p25 protein, deemed to be encoded by the DNA sequence, extending from about nucleotide 656 up to about nucleotide 1300 of LAVcDNA;

—a p13 protein, deemed to be encoded by the DNA sequence, extending from about nucleotide 1371 to about nucleotide 1650;

—a p18 protein, deemed to be encoded by the DNA sequence, extending from about nucleotide 336 up to about nucleotide 611.

More particularly the invention relates to monoclonal antibodies recognizing polypeptides having peptidic sequences identical or equivalent to those encoded by the DNA sequences extending approximately between the following nucleotide positions:

— 336 to 1650 (p55)
— 336 to 611 (p18)
— 13171 to 1650 (p13)
— 656 to 1300 (p25).

It should be mentioned that the p13, p18 and p25 all appear to derive from a same precursor, i.e. p55.

More particularly the invention concerns the monoclonal antibodies produced by the hybridomas deposited on October 24, 1984 at the "Collection Nationale des Cultures de Microorganismes" or, under the abbreviated form "CNCM", under the numbers which follow:

| | |
|---|---|
| LAV-A1 (p18) | n° I-335 |
| LAV-B1 (p25) | n° I-356 |
| LAV-C1 (p25) | n° I-357 |
| LAV-D1 (membrane) | n° I-358 |
| LAV-E1 (p25) | n° I-359 |
| LAV-F1 (p13) | n° I-360. |

The designations used for identifying the hybridomas correspond to the purified peptides obtained by standard purification procedures starting from lysates of LAV virus, which peptides were initially used for immunizing the animals from which the splenic cells used for the production of the corresponding hybridomas were obtained. Purified peptides for use in the production of said monoclonal antibodies can also be obtained by immunizing animals with the corresponding purified expression products of the DNA recombinants disclosed in the abovemen- tioned PCT application and European application. The general procedure for making said hybridomas will be described later.

A general procedure used for the production of each of the above said monoclonal antibodies will be described hereafter.

Immunisation of mice

Groups of 6—8 weeks old Balb/c mice were used. The different groups received the different proteins mentioned hereabove respectively. The immunization protocols, identical in all groups, comprised injections three times by the intraperitoneal route, then once by the intravenous route, each time of 10 µg of the antigenic preparation in the presence of Freund complete adjuvant at day 0, and of incomplete Freund adjuvant at day 14 without adjuvant at days 28 and 42.

Fusion and culture of the hybrids

The azaguanine-resistant and non secretor variant 6.53 of myeloma P3×63 Ag8, which itself originated from the MPOC-21 cell line was used. The fusion with the splenocytes of the immunized mice was performed in the presence of polyethylene-glycol 4000, according to the technique of FASEKAS DE ST-GROTH and SCHEIDEGGER at day 45. The selection of the hybrides in RPMI 16—40 "HAT" medium was carried out according to the same culture technique in plates comprising 24 wells (Costar).

The hybridomas which produced the specific antibodies were then cloned in plates comprising 96 wells respectively, in the presence of a feeder layer of syngenic thymocytes. The secreting clones selected were then expanded in plates comprising 24 wells respectively, still in the presence of thymocytes. When confluence appeared in one of the wells, the clone was intraperitoneally injected to BALB/c mice which had received 8 days earlier an injection of Pristane and/or maintained in liquid culture.

Detection of the anti-LAV antibodies

Five different techniques have permitted the characterisation of clones producing the antibodies of desired specificity. In a first step, the hybrids secreting the desired antibodies were detected by an ELISA assay that revealed the mice immunoglobulins in the supernatant. Starting from this first selection, the supernatants which contained antibodies oriented against the viral constituents sought were screened by means of an ELISA assay or by an immunogluorescence assay on human cells that produced the virus. Finally the supernatants were analyzed by radioimmunoprecipitation of virus labelled with $^{35}$S-cysteine and by the Western-Blot technique on a viral preparation whereby the specificities of the anti-LAV antibodies were determined.

Results

The cells obtained, starting from different fusions were then cultivated in 648 wells. The microscopic examination has shown that most of these wells contained a single hybrid clone capable of growing in the "HAT" selective medium. More than 50% of the clones produced antibodies that provided a positive response in the antivirus ELISA assay. The most representative fusions were tested by the Western-Blot technique and several hybridomas of each group were subcloned, taking into account their specificity, their reactivity in the antivirus ELISA assay and their development rate in cultures. Hybrids were retained which produced antibodies which recognized more specifically the proteins or polypeptides which had been used initially for immunizing the mice. All sub-clones obtained were shown to secrete antibodies which after expression, were injected in syngenic mice. The analysis of antibody specificities in the different ascites liquids obtained confirmed the specificities of the antibodies formed in each of the ascites with respect to the corresponding proteins.

The hybridomas which have been deposited at the CNCM and which were identified above are representative of the hybridomas that can be obtained using the above proteins. They form part of the invention too.

The monoclonal antibodies obtained can themselves be brought into play for purifying proteins or polypeptides which have in common an antigenic site with the proteins initially used for producing the hybridomas. The invention thus also relates to the purification processes per se. Such processes are advantageously used for the treatment of lysates of LAV, of infected T lymphocytes or of any other cells capable of producing LAV or an analogous virus. This process can also be applied to the identification of the proteins produced by cells which have been genetically engineered with recombinant DNAs as defined above and containing a DNA sequence encoding the relevant epitope. The monoclonal antibodies used in said process are advantageously immobilized on a solid support, for instance one suitable for affinity chromatography operations, such as a tri-dimensional cross-linked agarose lattice, commercialized under the trademark SEPHAROSE by the Swedish Company PHARMACIA A.G., for instance by the cyanogene bromide method.

The process of the invention thus comprises the step including contacting the solution containing said polypeptide with an affinity column carrying said monoclonal antibodies in order to selectively retain said polypeptides, then recovering the polypeptide upon dissociation of the antigen-antibody complex by means of an appropriate buffer, for instance a salt solution of appropriate ionic strength, for instance at pH 2—4. A suitable salt for constituting such buffers is formed of ammonium acetate.

Having isolated such polypeptide it will immediately appear that the same monoclonal antibodies can be further used for the study of fragments obtained from the corresponding polypeptide likely to contain the relevant epitope, said fragments having been obtained from the larger polypeptide, for instance by cleavage of the latter by enzymes capable of fragmenting polypeptides or proteins. By way of examples of such enzymes, one may mention the enzyme of *Staphylococcus aureus* V 8, alphachymotrypsin, the mouse submaxillary gland protease commercialized by the BOEHRINGER Company, the collagenase *Vibrio alginolyticus chemovar iophagus*, which recognizes specifically Gly-Pro and Gly-Ala dipeptides, etc.

The monoclonal antibody produced by the hybridoma deposited at the CNCM under Nr. I-355 is of particular significance. More particularly the monoclonal antibodies recognize both p18 and p55. Consequently it follows that the epitope more significantly recognized by said antibody remains unmodified when p55 (precursor of p18 and p25) is cleaved into its different components.

Thus this antibody is of particular interest for purifying both p18 and p55. The other components included in p55, particularly p25 can then be purified starting from the purified p55. It has been further found that said antibody is capable of recognizing the LAV virus in compositions containing same. It can be hypothesized that p18 behaves accordingly as a transmembranous protein, which is at least particularly exposed through the virus envelope and which is further expressed by the cells.

This antibody is thus of particular interest for
—the detection of viral particles in a biological sample, particularly a serum obtained from patients to be diagnosed for AIDS or LAS,
—the detection of infected lymphocytes,
—the detection in a biological sample as mentioned above or in a culture of infected lymphocytes,
—the treatment by the antibody of cells which express the virus.

The invention further relates
—to any other monoclonal antibody which recognizes any of the epitopes more specifically recognized by the monoclonal antibodies secreted by the hybridomas which have been deposited at the CNCM and, accordingly,
—to the hybrodomas which secrete said other monoclonal antibodies.

## Claims

1. Monoclonal antibodies which recognize a core protein of the lymphadenopathy associated-virus (LAV) characterized in that it is selected from the group of the monoclonal antibodies which are secreted by the hybridomas deposited at the CNCM under numbers I-355, I-356, I-357, I-358, I-359 and I-360 or any monoclonal antibody recognizing the same epitope as that recognized by a monoclonal antibody produced by any of the above mentioned hybridomas.

2. The monoclonal antibodies of claim 1 which recognize a p13 protein of LAV.

3. The monoclonal antibodies of claim 1 which recognize a p18 protein of LAV.

4. The monoclonal antibodies of claim 1 which recognize a p25 protein of LAV.

5. The monoclonal antibodies of claim 1 which recognize a p55 protein of LAV.

6. The monoclonal antibodies of claim 1 which recognize both a p18 protein and a p55 protein of LAV.

7. The hybridomas which secrete the monoclonal antibodies of any of claims 1 to 6.

8. The use of a monoclonal antibody according to any of claims 1 to 6 for the *in vitro* detection of the corresponding polypeptides in a biological sample or of the corresponding expression products on LAV-infected lymphocytes.

9. A method for the purification of a polypeptide contained in solubilized form in a biological sample, which polypeptide contains an epitope recognized by one of the monoclonal antibodies according to any of claims 1 to 6, wherein said method comprises contacting said biological medium with the corresponding monoclonal antibody affixed to or immobilized on an insolubilized support for causing the fixation of said polypeptide on said immobilized monoclonal antibody, whereby a polypeptide-antibody complex is formed, separating the non-fixed polypeptides and recovering said fixed antibody by the dissociation of said polypeptide-antibody complex.

## Patentansprüche

1. Monoklonale Antikörper, die ein Kernprotein des Lymphadenopathie-assoziierten Virus (LAV) erkennen, dadurch gekennzeichnet, daß sie ausgewählt sind aus der Gruppe der monoklonalen Antikörper, die durch die Hybridomen abgesondert werden, die bei der CNCM unter den Nummern I-355, I-356, I-357, I-358, I-359 und I-360 hinterlegt sind, oder jeder monoklonale Antikörper, der das gleiche Epitop erkennt wie dasjenige, das durch einen durch eines der oben genannten Hybridomen hergestellten monoklonalen Antikörper erkannt wird.

2. Monoklonale Antikörper nach Anspruch 1, die ein p13-Protein von LAV erkennen.

3. Monoklonale Antikörper nach Anspruch 1, die ein p18-Protein von LAV erkennen.

4. Monoklonale Antikörper nach Anspruch 1, die ein p25-Protein von LAV erkennen.

5. Monoklonale Antikörper nach Anspruch 1, die ein p55-Protein von LAV erkennen.

6. Monoklonale Antikörper nach Anspruch 1, die sowohl ein p18-Protein als auch ein p55-Protein von LAV erkennen.

7. Hybridome, die die monoklonalen Antikörper nach einem der Ansprüche 1 bis 6 absondern.

8. Die Verwendung eines monoklonalen Antikörpers nach einem der Ansprüche 1 bis 6 für die *in vitro*-Bestimmung der entsprechenden Polypeptide in einer biologischen Probe oder der entsprechenden Expressionsprodukte auf LAVinfizierten Lymphocyten.

9. Verfahren zur Reinigung eines in löslicher Form in einer biologischen Probe enthaltenen Polypeptids, das ein Epitop enthält, das durch einen der monoklonalen Antikörper nach einem der Ansprüche 1 bis 6 erkannt ist, das darin besteht, daß das biologische Medium mit dem entsprechenden monoklonalen Antikörper, der an einem unlösbaren Träger befestigt oder blockiert ist, um die Fixierung des Polypeptids auf dem blockierten monoklonalen Antikörper zu bewirken, wodurch ein Polypeptid-Antikörper-Komplex gebildet wird, kontaktiert wird, die nicht-fixierten Polypeptide abgetrennt werden und der fixierte Antikörper durch die Spaltung des Polypeptid-Antikörper-Komplexes gewonnen wird.

**Revendications**

1. Anticorps monoclonaux reconnaissant une nucléoprotéine du virus associé à la lymphadéno-pathie (LAV) caractérisés en ce qu'ils sont sélectionnés parmi le groupe des anticorps monoclonaux sécrétés par les hybridomes déposés à la CNCM sous les numéros I-355, I-356, I-357, I-358, I-359 et I-360, ou tout anticorps monoclonal reconnaissant le même épitope que celui reconnu par l'anticorps monoclonal produit par l'un des hybridomes ci-dessus mentionnés.

2. Anticorps monoclonaux selon la revendication 1, qui reconnaissent une protéine p13 de LAV.

3. Anticorps monoclonaux selon la revendication 1, qui reconnaissent une protéine p18 de LAV.

4. Anticorps monoclonaux selon la revendication 1, qui reconnaissent une protéine p25 de LAV.

5. Anticorps monoclonaux selon la revendication 1, qui reconnaissent une protéine p55 de LAV.

6. Anticorps monoclonaux selon la revendication 1, qui reconnaissent à la fois une protéine p18 et une protéine p55 de LAV.

7. Hybridomes secréteurs des anticorps monoclonaux selon l'une des revendications 1 à 6.

8. Utilisation d'un anticorps monoclonal selon l'une des revendications 1 à 6 pour la détection *in vitro* des polypeptides correspondants dans un échantillon biologique ou des produits d'expression correspondants sur des lymphocytes infectés par LAV.

9. Méthode pour la purification d'un polypeptide contenu sous une forme solubilisée dans un échantillon biologique, lequel polypeptide contient un épitope reconnu par l'un des anticorps monoclonaux selon l'une des revendications 1 à 6, ladite méthode comprenant le contact dudit milieu biologique avec l'anticorps monoclonal correspondant fixé ou immobilisé sur un support insoluble pour effectuer la fixation dudit polypeptide sur ledit anticorps monoclonal immobilisé, permettant la formation d'un complexe polypeptide-anticorps, séparation des polypeptides non fixés et récupération dudit anticorps fixé par dissociation dudit complexe polypeptides-anticorps.